(19) Europäisches Patentamt
European Patent Office
Office européen des brevets

(11) **EP 4 756 018 A1**

(12) **EUROPEAN PATENT APPLICATION**
published in accordance with Art. 153(4) EPC

(43) Date of publication:
**10.06.2026 Bulletin 2026/24**

(21) Application number: **25794955.2**

(22) Date of filing: **02.04.2025**

(51) International Patent Classification (IPC):
**C12N 9/10** (2006.01)  **C12N 15/77** (2006.01)
**C12P 13/10** (2006.01)

(52) Cooperative Patent Classification (CPC):
**C12N 9/10; C12N 15/77; C12P 13/10**

(86) International application number:
**PCT/KR2025/004328**

(87) International publication number:
**WO 2025/225912 (30.10.2025 Gazette 2025/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **25.04.2024  KR 20240055639**

(71) Applicant: **CJ Cheiljedang Corporation
Seoul 04560 (KR)**

(72) Inventors:
• **HAN, Seunghee
Seoul 04560 (KR)**
• **LEE, Ji Yeon
Seoul 04560 (KR)**

• **PARK, So-Yeon
Seoul 04560 (KR)**
• **PARK, Sung Eun
Seoul 04560 (KR)**
• **LEE, Zeewon
Seoul 04560 (KR)**
• **KIM, Bina
Seoul 04560 (KR)**

(74) Representative: **Meissner Bolte Partnerschaft
mbB
Patentanwälte Rechtsanwälte
Postfach 86 06 24
81633 München (DE)**

Remarks:
The complete document including Reference
Table(s) and the Sequence Listing(s) can be
downloaded from the EPO website

(54) **NOVEL BIFUNCTIONAL GLUTAMATE N-ACETYLTRANSFERASE/AMINO-ACID
ACETYLTRANSFERASE VARIANT, AND METHOD FOR PRODUCING GLUTAMATE-BASED
AMINO ACIDS USING SAME**

(57)  The present disclosure relates to a novel bifunctional glutamate N-acetyltransferase/amino-acid acetyltransferase variant, a Corynebacterium sp. microorganism comprising the variant, and a method for producing glutamate-based amino acids, comprising the step of culturing the microorganism.

Processed by Luminess, 75001 PARIS (FR)

**Description**

[TECHNICAL FIELD]

Cross-reference to related application(s)

The present application claims the benefit of the priority based on Korean Patent Application No. 10-2024-0055639 filed on April 25, 2024, and the entire contents disclosed in the documents of the corresponding Korean patent application are incorporated as a part in the present description.

The present disclosure relates to a novel bifunctional glutamate N-acetyltransferase/amino acid acetyltransferase variant and a method for producing glutamate family amino acids using the same.

[BACKGROUND ART]

Glutamate is one of the proteinogenic amino acids widely found in plants, animals, and microorganisms, and is metabolized in vivo by being converted into ornithine (L-ornithine), citrulline (L-citrulline), arginine (L-arginine), putrescine, and the like.

Ornithine has been used as a nutritional supplement due to its effects on muscle formation and reduction of body fat, and is also used as a pharmaceutical agent for improving liver cirrhosis and hepatic dysfunction. Citrulline is known to have physiological activities, such as improvement of blood flow, reduction of blood pressure, neurotransmission, enhancement of immune function, scavenging of reactive oxygen species, and the like, resulting from promotion of ammonia metabolism or vasodilation. In addition, arginine is used for pharmaceutical purposes, such as a liver function enhancer, a brain function enhancer, a comprehensive amino acid preparation, and the like, and is also used for food, such as a fish paste additive, a health beverage additive, a salt substitute for patients with hypertension, and the like. Putrescine is a type of polyamine found in a wide range of organisms from bacteria to plants and animals, and plays an important role in cell proliferation and normal cell growth, and is also known to be an important substance in defense mechanisms against oxidative stress.

Microorganisms of the genus *Corynebacterium*, in particular *Corynebacterium glutamicum*, are Gram-positive microorganisms that are widely used for amino acid production. For production of amino acids, target substance-specific approaches such as increasing the expression level of genes encoding enzymes involved in biosynthesis of amino acids or removing genes unnecessary for biosynthesis of amino acids have mainly been used in strains of the genus *Corynebacterium* (US 9644009 B2).

With the increasing demand for glutamate family amino acids, a need for research on methods capable of efficiently producing glutamate family amino acids has emerged.

[DISCLOSURE]

[TECHNICAL PROBLEM]

One embodiment of the present disclosure provides a bifunctional glutamate N-acetyltransferase/amino acid acetyltransferase (Bifunctional glutamate N-acetyltransferase/aminoacid acetyltransferase ArgJ) variant in which, from the N-terminus in the amino acid sequence of SEQ ID NO: 1, the amino acid corresponding to the 201st amino acid is substituted with phenylalanine (Phe or F), and the amino acid corresponding to the 355th amino acid is substituted with glutamic acid (Glu or E).

Another embodiment of the present disclosure provides a polynucleotide encoding the variant.

Other embodiment of the present disclosure provides a recombinant vector comprising the polynucleotide.

Other embodiment of the present disclosure provides a microorganism of the genus *Corynebacterium*, comprising the variant, a polynucleotide encoding the variant, or a recombinant vector comprising the polynucleotide. The microorganism of the genus *Corynebacterium* may have a production ability of glutamate family amino acids, or have an increased glutamate family amino acids production ability as compared with a non-modified microorganism of the genus *Corynebacterium.*

Other embodiment of the present disclosure provides a method for producing glutamate family amino acids, comprising culturing a microorganism of the genus *Corynebacterium* comprising the variant, a polynucleotide encoding the variant, or a recombinant vector comprising the polynucleotide, and recovering glutamate family amino acids from the cultured microorganism, medium, or both of them.

Other embodiment of the present disclosure provides a use for producing glutamate family amino acids, of a microorganism of the genus *Corynebacterium*, comprising the variant, a polynucleotide encoding the variant, or a recombinant vector comprising the polynucleotide,.

[TECHNICAL SOLUTION]

This will be described in detail as follows. Meanwhile, each description and embodiment disclosed in the present disclosure may be applied to each other description and embodiment. In other words, all combinations of the various elements disclosed in the present disclosure fall within the scope of the present disclosure. In addition, the scope of the present disclosure should not be construed as being limited by the specific descriptions described below. Further, a person having ordinary skill in the art may recognize or ascertain a number of equivalents to the specific aspects of the present disclosure described herein using only routine experimentation. Furthermore, such equivalents are intended to be included in the present disclosure.

In addition, throughout the present disclosure, a number of papers and patent documents are referred and citations thereof are represented. The disclosed contents of the cited papers and patent documents are incorporated herein by reference in their entirety, thereby more clearly explaining the level of the technical field to which the present disclosure pertains and the contents of the present disclosure.

One embodiment of the present disclosure provides a bifunctional glutamate N-acetyltransferase/amino acid acetyltransferase variant in which, from the N-terminus in the amino acid sequence of SEQ ID NO: 1, the amino acid corresponding to the 201st amino acid is substituted with phenylalanine (Phe, or F), and the amino acid corresponding to the 355th amino acid is substituted with glutamic acid (Glu, or E).

The amino acid corresponding to the 201st amino acid from the N-terminus in the amino acid sequence of SEQ ID NO: 1 may be isoleucine (Ile, or I), and the amino acid corresponding to the 355th amino acid is aspartic acid (Asp, or D).

The bifunctional glutamate N-acetyltransferase/amino acid acetyltransferase variant of the present disclosure may comprise the amino acid sequence of SEQ ID NO: 3, and may consist of (be composed of) the amino acid sequence.

In addition, in the variant of the present disclosure, in the amino acid sequence set forth in SEQ ID NO: 3, based on the amino acid sequence of SEQ ID NO: 1, the amino acid corresponding to position 201 may be phenylalanine, and the amino acid corresponding to position 355 may be glutamic acid, and the variant may comprise an amino acid sequence having at least 70%, 75%, 80%, 85%, 90%, 95%, 96%, 97%, 98%, 99%, 99.5%, 99.7% or 99.9% or higher homology or identity with the amino acid sequence set forth in SEQ ID NO: 3. Furthermore, it is apparent that as long as it is an amino acid having such homology and identity and exhibiting an efficacy corresponding to that of the variant of the present disclosure, even variants having an amino acid sequence in which some sequences are deleted, modified, substituted, conservatively substituted, or added are included in the scope of the present disclosure.

For example, it is a case in that it has an addition or deletion of a sequence that does not alter the function of the variant of the present disclosure, a naturally occurring mutation, a silent mutation, or a conservative substitution at the N-terminus, C-terminus, and/or inside of the amino acid sequence.

The term "conservative substitution" refers to substitution of one amino acid with another amino acid having similar structural and/or chemical properties. Such amino acid substitutions may generally occur based on similarities in polarity, charge, solubility, hydrophobicity, hydrophilicity, and/or amphipathic nature of residues. In common, conservative substitution may hardly affect or not affect activity of protein or polypeptide.

In the present disclosure, the term, "variant" refers to a protein or a gene encoding it in which at least one amino acid is different from a sequence defined by a certain sequence number for conservative substitution and/or modification, but the functions or properties of the protein are maintained. Such a variant may be generally identified by modifying at least one amino acid among the amino acid sequence of the protein, and evaluating properties of the modified protein. In other words, the ability of the variant may be increased, unchanged, or decreased as compared to that of the polypeptide before mutation. In addition, some variants may include variants in which one or more parts such as an N-terminal leader sequence or a transmembrane domain are removed. Other variants may include variants in which a part is removed from the N-terminus and/or C-terminus of mature protein. The term "variant" may be used interchangeably with terms such as a mutant, modification, mutant polypeptide, mutated protein, mutation and variant, and the like (including as modification, modified polypeptide, modified protein, mutant, mutein, divergent, etc. as English expressions), and is not limited thereto as long as the term is used to have a meaning of being mutated. For the purposes of the present disclosure, the variant may be a polypeptide comprising an amino acid sequence set forth in SEQ ID NO: 3, in which the amino acid corresponding to the 201th amino acid is substituted with phenylalanine, and the amino acid corresponding to the 355th amino acid is substituted with glutamic acid, of the amino acid sequence of SEQ ID NO: 1.

Furthermore, the variant may comprise deletion or addition of amino acids having minimal effects on the properties and secondary structure of the polypeptide. For example, a signal (or leader) sequence involved in protein translocation co-translationally or post-translationally may be conjugated at the N-terminus of the variant. In addition, the variant may be conjugated with another sequence or linker to be identified, purified, or synthesized.

In order to describe the variants provided in the present disclosure, the following nomenclature is used.

In the present disclosure, referring to a specific position of an amino acid sequence may include referring to an amino acid that is present at that position or is substituted at that position. Referring to an amino acid at a specific position may be described in various ways. For example, "position 201" may be described as "amino acid at position 201" or "the 201st amino acid." In addition, for example, when the amino acid at the 201st position is isoleucine, it may be described as "I" or "Ile201."

An amino acid substitution may be expressed by describing, in order, the amino acid prior to substitution, the position, and the substituted amino acid. The amino acids may be expressed using conventional one-letter and three-letter codes. As one example, when isoleucine, which is an amino acid at position 201 of a specific sequence, is substituted with phenylalanine, it may be described as "I201F" or "Ile201Phe".

Multiple mutations may be described using "+". For example, the description such as "I201F + D355E" means that isoleucine, which is an amino acid at position 201, is substituted with phenylalanine, and aspartic acid, which is an amino acid at position 355, is substituted with glutamic acid.

In the present disclosure, 'homology' or 'identity' refers to the degree of similarity between two given amino acid sequences or base sequences, and may be expressed as a percentage. The terms homology and identity may be often used interchangeably.

The sequence homology or identity of a conserved polynucleotide or polypeptide may be determined by a standard alignment algorithm, and a default gap penalty established by the used program may be used together. Substantially, homologous or identical sequences may be generally hybridized with all or part of the sequences under mild or highly stringent conditions. It is obvious that hybridization also includes hybridization with a polynucleotide containing common codons or codons considering codon degeneracy.

Whether any two polynucleotide or polypeptide sequences have homology, similarity or identity, may be determined, for example, using a known computer algorithm such as "FASTA" program using a default parameter such as Pearson et al (1988) [Proc. Natl. Acad. Sci. USA 85]: 2444. Otherwise, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version), (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, using BLAST or ClustalW of National Center for Biotechnology Information database, homology, similarity, or identity may be determined.

The homology, similarity or identity of polynucleotides or polypeptides may be determined by comparing sequence information using for example, GAP computer program such as Needleman et al. (1970), J Mol Biol. 48:443, known in for example, Smith and Waterman, Adv. Appl. Math (1981) 2:482. In summary, GAP program may be defined as a value of dividing the total number of symbols in the shorter of two sequences by the number of similarly aligned symbols (i.e., nucleotides or amino acids). Default parameters for the GAP program may include (1) a binary comparison matrix (containing values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix), disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or gap opening penalty 10, gap extension penalty 0.5); and (3) no penalty for end gaps.

As one example of the present disclosure, the variant of the present disclosure may have bifunctional glutamate N-acetyltransferase/amino acid acetyltransferase activity. In addition, the variant may have activity that increases glutamate family amino acids production ability as compared to w a wild-type polypeptide.

In the present disclosure, the term, "bifunctional glutamate N-acetyltransferase/amino acid acetyltransferase (bifunctional glutamate N-acetyltransferase/amino-acid acetyltransferase ArgJ)", refers to an enzyme having both a glutamate N-acetyltransferase activity that converts glutamate and acetyl-CoA into N-acetylglutamate, and an amino acid acetyltransferase activity that converts acetyl-L-ornithine and L-glutamate into L-ornithine and N-acetyl-L-glutamate. The bifunctional glutamate N-acetyltransferase/amino acid acetyltransferase of the present disclosure may be used interchangeably with arginine biosynthesis bifunctional protein ArgJ, or ArgJ protein. The "glutamate N-acetyltransferase" may be used interchangeably with the terms N2-acetyl-L-ornithine: L-glutamate N-acetyltransferase (N2-acetyl-L-ornithine: L-glutamate N-acetyltransferase), ornithine acetyltransferase, ornithine transacetylase, and acetylornithine glutamate acetyltransferase, and the "amino acid acetyltransferase" may be used interchangeably with the terms acetyl-CoA:L-glutamate N-acetyltransferase, and N-acetylglutamate synthetase. In the present disclosure, the sequence of the bifunctional glutamate N-acetyltransferase/amino acid acetyltransferase may be obtained from a publicly known database, namely GenBank of NCBI (for example, WP_040967390.1). Specifically, it may be a polypeptide having activity of bifunctional glutamate N-acetyltransferase/amino acid acetyltransferase encoded by the *argJ* gene, but is not limited thereto.

In the present disclosure, the term "glutamate family amino acids" refers to amino acids that can be biosynthesized using glutamate as a precursor. Specifically, the glutamate family amino acids may be at least one selected from the group consisting of L-ornithine, L-citrulline, L-arginine and putrescine, but are not limited thereto as long as the amino acids can be biosynthesized using glutamate as a precursor. The "glutamate family amino acids" of the present disclosure may be used interchangeably with "glutamic acid family amino acids".

In the present disclosure, the term, "corresponding to" refers to an amino acid residue at a position listed in a polypeptide, or an amino acid residue similar or identical or homologous to a residue listed in a polypeptide. Confirming an amino acid at a corresponding position may determine a specific amino acid of a sequence referring to a specific sequence. The "corresponding region" used in the present disclosure generally refers to a similar or corresponding position in a related protein or reference protein.

For example, any amino acid sequence may be aligned with SEQ ID NO: 1, and based thereon, each amino acid residue of the amino acid sequence may be numbered with reference to the numerical position of the corresponding to the amino acid residue of SEQ ID NO: 1. For example, the sequence alignment algorithm as described in the present disclosure, may confirm a location of amino acids, or a location where modification such as substitution, insertion or deletion or the like occurs, compared to a query sequence (also called "reference sequence").

For this alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277) and the like may be used, but not limited thereto, and a sequence alignment program known in the art, pairwise sequence comparison algorithm and the like may be appropriately used.

Another aspect of the present disclosure provides a polynucleotide encoding the protein variant of the present disclosure.

In the present disclosure, the term, "polynucleotide" refers to a DNA or RNA strand of a certain length or more, which is a polymer of nucleotides in which nucleotide monomers are connected in a long chain form by covalent bonds, and more specifically, a polynucleotide fragment encoding the variant.

The polynucleotide encoding the variant of the present disclosure may include a base sequence encoding the amino acid sequence set forth in SEQ ID NO: 3. As one example of the present disclosure, the polynucleotide of the present disclosure may have or comprise the sequence of SEQ ID NO: 4. In addition, the polynucleotide of the present disclosure may consist of, or be essentially composed of, the sequence of SEQ ID NO: 4. In another example, the polynucleotide of the present disclosure may comprise a nucleic acid sequence having at least 70%, 75%, 80%, 85%, 90%, 95%, 96%, 97%, 98%, 99%, 99.5%, 99.7% or 99.9% or more homology or identity with the nucleic acid sequence set forth in SEQ ID NO: 4, in which the base corresponding to position 601 is T, and the base corresponding to position 1065 is G, based on the nucleic acid sequence of SEQ ID NO: 2 in the nucleic acid sequence set forth in SEQ ID NO: 4. In addition, it is obvious that even a polynucleotide having a nucleotide sequence in which some sequences are deleted, modified, substituted, conservatively substituted, or added is included in the scope of the present disclosure, as long as it is a sequence which has such homology or identity and shows efficacy corresponding to the variant of the present disclosure.

The polynucleotide of the present disclosure may have various modifications in coding regions within a range which does not change the amino acid sequence of the variant of the present disclosure, in consideration of degeneracy of codons or codons preferred in an organism in which the variant of the present disclosure is to be expressed. Specifically, the polynucleotide of the present disclosure may have or comprise a base sequence with homology or identity of 70% or more, 75% or more, 80% or more, 85% or more, 90% or more, 95% or more, 96% or more, 97% or more, 98% or more, and less than 100% to the sequence of SEQ ID NO: 4, or consist of or essentially consist of a base sequence with homology or identity of 70% or more, 75% or more, 80% or more, 85% or more, 90% or more, 95% or more, 96% or more, 97% or more, 98% or more, and less than 100% to the sequence of SEQ ID NO: 4, but is not limited thereto. In this case, in a sequence having the homology or identity described above, a codon encoding the amino acid corresponding to position 201 of SEQ ID NO: 3 may be one of the codons encoding phenylalanine, and a codon encoding the amino acid corresponding to position 355 may be one of the codons encoding glutamic acid.

In addition, the polynucleotide of the present disclosure may comprise a probe that can be prepared from a known gene sequence, for example, any sequence capable of hybridizing with a complementary sequence to all or a part of the polynucleotide sequence of the present disclosure under stringent conditions without limitation. The "stringent condition" means a condition that allows specific hybridization between polynucleotides. These conditions are specifically described in the documents (See J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, a condition of hybridizing polynucleotides with high homology or identity, polynucleotides with homology or identity of 70% or more, 75% or more, 80% or more, 85% or more, 90% or more, 95% or more, 96% or more, 97% or more, 98% or more, or 99% or more, and not hybridizing polynucleotides with homology or identity lower than that, or a condition of washing at a salt concentration and temperature corresponding to 60°C, 1×SSC, 0.1% SDS, specifically, 60°C, 0.1×SSC, 0.1% SDS, more specifically, 68°C, 0.1×SSC, 0.1% SDS, which is a washing condition of common southern hybridization, once, specifically, twice to 3 times may be listed.

Hybridization requires that two nucleotides have complementary sequences, but mismatches between bases may be allowed depending on the stringency of hybridization. The term, "complementary" may be used to describe relationship between nucleotide bases capable of hybridizing to each other. For example, in case of DNA, adenosine is complementary to thymine, and cytosine is complementary to guanine. Therefore, the polynucleotide of the present disclosure may also comprise not only a substantially similar nucleotide sequence, but also an isolated nucleic acid fragment complementary to the entire sequence.

Specifically, the polynucleotide with homology or identity to the polynucleotide of the present disclosure may be detected using a hybridization condition including a hybridization step at a Tm value of 55°C and using the afore-mentioned conditions. In addition, the value of Tm may be 60°C, 63°C or 65°C, but not limited thereto, and may be appropriately adjusted by those skilled in the art according to the purpose.

The suitable stringency to hybridize the polynucleotide depends on the length and degree of complementarity of the polynucleotide, and variables are well known in the art (For example, J. Sambrook et al., as described above).

Another aspect of the present disclosure provides a vector comprising the polynucleotide of the present disclosure. The vector may be an expression vector to express the polynucleotide in a host cell, but not limited thereto.

In the present disclosure, the "vector" may comprise a DNA product comprising a base sequence of a polynucleotide encoding the target polypeptide operably linked to an expression regulatory region (or expression regulatory sequence) suitable for expressing a target polypeptide in an appropriate host. The expression regulatory region may comprise a promoter that can initiate transcription, any operator sequence to regulate such transcription, a sequence encoding an appropriate mRNA ribosome binding site, and/or a nucleic acid sequence that regulates termination of transcription and translation. The vector may be transformed into a suitable host cell, and then be replicated or function independently of the host genome, and be integrated into the genome itself.

The vector used in the present disclosure is not particularly limited, and any vector known in the art may be used. Examples of the commonly used vector may include plasmids, cosmids, viruses, and bacteriophages in a natural state or a recombined state. For example, as the phage vector or cosmid vector, pWE15, M13, MBL3, MBL4, IXII, ASHII, APII, t10, t11, Charon4A, and Charon21A, and the like may be used, and as the plasmid vector, pDZ-based, pBR-based, pUC-based, pBluescriptII-based, pGEM-based, pTZ-based, pCL-based and pET-based and the like may be used. Specifically, pDZ, pDC, pDCM2, pDC24, pACYC177, pACYC184, pCL, pECCG117, pUC19, pBR322, pMW118, pCC1BAC, pCES208 vector, and the like may be used.

As one example, through a vector for inserting chromosome into a cell, a polynucleotide encoding a target polypeptide may be inserted into chromosome. Insertion of the polynucleotide into chromosome may be performed by any method known in the art, for example, homologous recombination, but not limited thereto. A selection marker to confirm whether the chromosome is inserted may be further comprised. The selection marker is to select a cell transformed with a vector, that is, to confirm whether a target nucleic acid molecule is inserted, and markers which impart selectable phenotypes such as drug resistance, auxotrophy, resistance to a cytotoxic agent, or expression of a surface protein may be used. In an environment treated with a selective agent, only cells expressing the selection marker survive or show other phenotypes, so transformed cells may be selected.

In the present disclosure, the term, "transformation" means introducing a vector comprising a polynucleotide encoding a target polypeptide into a host cell or microorganism so that the polypeptide encoded by the polynucleotide is to be expressed in the host cell. As long as the transformed polynucleotide can be expressed in a host cell, all of them may be included regardless of whether they are inserted into chromosome of the host cell or located outside the chromosome. In addition, the polynucleotide comprises DNA and/or RNA encoding a target polypeptide. The polynucleotide may be introduced in any form, as long as it can be introduced into a host cell and expressed. For example, the polynucleotide may be introduced into a host cell in a form of expression cassette, which is a gene structure comprising all elements required for being expressed by itself. The expression cassette may commonly comprise a promoter operably linked to the polynucleotide, a transcription termination signal, a ribosome binding site and a translation termination signal. The expression cassette may be in a form of an expression vector capable of self-replication. In addition, the polynucleotide may be introduced into a host cell in its own form and be operably linked to a sequence required for expression in the host cell, but not limited thereto.

Furthermore, in the above, the term, "operably linked" refers to that the polynucleotide sequence is functionally linked to a promoter sequence which initiates and mediates transcription of the polynucleotide encoding the target variant of the present disclosure.

Another aspect of the present disclosure provides a microorganism of the genus *Corynebacterium*, comprising the variant of the present disclosure or the polynucleotide of the present disclosure.

The microorganism of the present disclosure may comprise the mutant polypeptide of the present disclosure, a polynucleotide encoding the polypeptide, or a vector comprising the polynucleotide of the present disclosure.

In the present disclosure, the term, "microorganism (or strain)" includes all of wild-type microorganisms or microorganisms in which genetic modification occurs naturally or artificially, and may be a microorganism of which specific mechanism is weakened or strengthened due to reasons such as insertion of a foreign gene or enhancement or inactivation of activity of an endogenous gene and the like, which is a microorganism comprising genetic modification for production of a target polypeptide, protein or product.

The microorganism of the present disclosure may be a microorganism comprising any one or more of the protein variant of the present disclosure, the polynucleotide of the present disclosure, and a vector comprising the polynucleotide of the present disclosure; a microorganism modified to express the variant of the present disclosure or the polynucleotide of the present disclosure; a microorganism (for example, recombinant strain) expressing the variant of the present disclosure, or the polynucleotide of the present disclosure; or a microorganism (for example, recombinant strain) having the activity of the variant of the present disclosure, but not limited thereto.

The microorganism of the present disclosure may be a microorganism having glutamate family amino acids production ability.

The microorganism of the present application may have enhanced glutamate family amino acids production ability.

The microorganism of the present application may be a microorganism that naturally has bifunctional glutamate N-acetyltransferase/amino acid acetyltransferase activity and/or glutamate family amino acids production ability, or a microorganism in which the variant of the present disclosure or a polynucleotide encoding the same (or a vector comprising the polynucleotide) is introduced into a parent strain that does not have bifunctional glutamate N-acetyltransferase/amino acid acetyltransferase activity or glutamate family amino acids production ability, and/or to which glutamate family amino acids production ability is imparted, but is not limited thereto.

As one example, the microorganism of the present disclosure is a cell or microorganism expressing the protein variant of the present disclosure, as transformed with a vector comprising the polynucleotide of the present disclosure, or a polynucleotide encoding the variant of the present disclosure, and for the purpose of the present disclosure, the microorganism of the present disclosure may include all microorganisms capable of producing glutamate family amino acids by comprising the variant of the present disclosure. For example, the microorganism of the present disclosure may be a natural wild-type microorganism or a recombinant strain with increased productivity of glutamate family amino acids by introducing the polynucleotide encoding the variant of the present disclosure into a natural wild-type microorganism or a microorganism producing glutamate family amino acids to express a bifunctional glutamate N-acetyltransferase/amino acid acetyltransferase variant.

The recombinant strain having increased glutamate family amino acids production ability may be a microorganism having increased glutamate family amino acids production ability as compared to a natural wild-type microorganism or a bifunctional glutamate N-acetyltransferase/amino acid acetyltransferase non-modified microorganism (that is, a microorganism expressing a wild-type (SEQ ID NO: 1) protein or a microorganism not expressing a variant (SEQ ID NO: 3) protein), but is not limited thereto. As an example, a strain to be compared for determining whether the glutamate family amino acids production ability is increased, which is a bifunctional glutamate N-acetyltransferase/amino acid acetyltransferase non-modified microorganism, may be a wild-type *Corynebacterium glutamicum* ATCC13869 strain, a *Corynebacterium glutamicum* ATCC13869 strain in which the activity of the ArgR protein and/or ArgF protein is weakened, a *Corynebacterium glutamicum* ATCC13869 strain in which the activity of the ArgR protein and/or ArgG protein is weakened, or a *Corynebacterium glutamicum* CJR100 strain (Korean Patent Application No. 10-2023-0048232), but is not limited thereto.

As one example, the recombinant strain with the increased production ability may have production ability of glutamate family amino acids increased by about 1% or more, about 2.5% or more, about 3% or more, about 3.1% or more, about 3.2% or more, about 3.3% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 20.5% or more, about 21% or more, about 21.5% or more, about 22% or more, about 22.5% or more, about 23% or more, about 23.5% or more, about 24% or more, about 24.5% or more, about 25% or more, about 25.5% or more, about 26% or more, about 26.5% or more, about 27% or more, about 27.5% or more, about 28% or more, about 28.5% or more, about 29% or more, about 29.5% or more, about 30% or more, about 31% or more, about 32% or more, about 33% or more, about 34% or more, or about 35% or more (the upper limit is not particularly limited, and for example, it may be about 300% or less, about 250% or less, or about 200% or less), as compared to a strain or non-modified microorganism before mutation (before introduction of a polynucleotide encoding the variant of the present disclosure), but is not limited thereto as long as it has an increase having a + value as compared to the production ability of the parent strain before mutation or non-modified microorganism. In another example, the recombinant strain with the increased production ability may have glutamate family amino acids production ability increased by about 1.02 folds or more, about 1.03 folds or more, about 1.05 folds or more, about 1.1 folds or more, about 1.12 folds or more, about 1.13 folds or more, 1.15 folds or more, 1.16 folds or more, 1.17 folds or more, 1.18 folds or more, 1.19 folds or more, about 1.2 folds or more, 1.25 folds or more, about 1.3 folds or more, or about 1.5 folds or more (the upper limit is not particularly limited, and for example, it may be about 10 folds or less, about 5 folds or less, about 4 folds or less, or about 3 folds or less), as compared to a parent strain before mutation or non-modified microorganism.

More specifically, the recombinant strain with the increased production ability may have L-ornithine production ability increased by about 1% or more, about 3% or more, about 4% or more, about 5% or more, about 6% or more, about 7% or more, about 7.5% or more, about 8% or more, about 9% or more, about 10% or more, about 12% or more, about 14% or more, about 15% or more, about 16% or more or about 17% or more (the upper limit is not particularly limited, and for example, it may be about 100% or less, about 50% or less, or about 30% or less), and may have L-citrulline production ability increased by about 1% or more, about 3% or more, about 4% or more, about 5% or more, about 6% or more, about 7% or more, about 8% or more, about 8.5% or more, about 10% or more, about 12% or more, about 14% or more, about 16% or more, about 18% or more, about 20% or more, about 22% or more, about 24% or more, about 26% or more or about 27% or more (the upper limit is not particularly limited, and for example, it may be about 100% or less, about 50% or less, or about 40% or less), and may L-arginine production ability increased by about 1% or more, about 2.5% or more, about 3% or more, about 3.1% or more, about 3.2% or more or about 3.3% or more (the upper limit is not particularly limited, and for example, it may be about 50% or less, about 40% or less, or about 30% or less), and may have putrescine production ability increased by about 1.3 folds or more, or about 1.5 folds or more (the upper limit is not particularly limited, and for example, it may be about 10 folds or less, about 5 folds or less, about 4 folds or less, or about 3 folds or less), as compared to a parent strain before mutation (before a polynucleotide encoding the variant of the present disclosure is introduced) or a non-modified microorganism.

The term "about" is a range including all of ±0.5, ±0.4, ±0.3, ±0.2, ±0.1, and the like, and includes all numerical values identical or similar to the numerical value appearing after the term of about, but is not limited thereto.

In the present disclosure, the term, "non-modified microorganism" does not exclude a strain comprising mutation that can occur naturally in a microorganism, and may mean a wild-type strain or natural strain itself, or a strain before traits are changed due to genetic mutation caused by natural or artificial factors. For example, the non-modified microorganism may mean a strain in which the bifunctional glutamate N-acetyltransferase/amino acid acetyltransferase variant described in the present disclosure is not introduced or before introduced. The "non-modified microorganism" may be interchangeably used with "strain before modification", "microorganism before modification", "non-mutated strain", "non-modified strain", "non-mutated microorganism" or "reference microorganism".

As another example of the present disclosure, the microorganism of the present disclosure may be a microorganism of the genus *Corynebacterium.* The microorganism of the genus *Corynebacterium* may be *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium pollutisoli*, *Corynebacterium imitans*, *Corynebacterium testudinoris* or *Corynebacterium flavescens*.

As another example of the present disclosure, the microorganism of the genus *Corynebacterium* having increased glutamate family amino acids production ability of the present disclosure, may be a microorganism in which the activity of at least a part of proteins in the glutamate family amino acids biosynthetic pathway is additionally enhanced, or the activity of at least a part of proteins in the glutamate family amino acids biosynthetic pathway is additionally weakened, thereby enhancing the glutamate family amino acids production ability.

Specifically, the microorganism of the present disclosure may be a microorganism mutated in order that the activity of ornithine carbamoyltransferase subunit F (ArgF) and/or arginine repressor (ArgR) are additionally weakened, or the *argF* gene and/or *argR* gene encoding the same are additionally deleted. In addition, the microorganism of the present disclosure may be a microorganism in which the activity of arginine repressor (ArgR) and/or argininosuccinate synthase (ArgG) is additionally weakened, or the *argR* gene and/or *argG* gene encoding the same are additionally deleted, and/or the activity of ornithine decarboxylase (ODC) is introduced. In addition, the microorganism of the present disclosure may be a microorganism mutated such that, in order to increase putrescine production ability, the activity of a protein encoded by the *NCgl1469* gene, which is an enzyme synthesizing N-acetyl putrescine of putrescine, is additionally weakened, or a gene encoding the same is deleted.

The amino acid sequences of ArgF, ArgR, ArgG, and ODC may be obtained from known databases such as Genebank of NCBI and the like. As one example, the amino acid sequence of ArgF of the present disclosure may comprise ANU33618.1 derived from *Corynebacterium glutamicum* ATCC13869 or an amino acid sequence having 90% or more homology thereto, and the amino acid sequence of ArgR may comprise ANU33619.1 derived from *Corynebacterium glutamicum* ATCC13869, or an amino acid sequence having 90% or more homology thereto, and the amino acid sequence of ArgG may comprise ANU33620.1 derived from *Corynebacterium glutamicum* ATCC13869, or an amino acid sequence having 90% or more homology thereto, and the amino acid sequence of ODC may comprise AAA64830.1 derived from *Lactobacillus* sp. 30A strain, or an amino acid sequence having 90% or more homology thereto, but is not limited thereto, and it is apparent that proteins having ArgF, ArgR, ArgG, or ODC activity derived from various organisms may be included.

In the present disclosure, the term, "weakening" of the polypeptide, is a concept including both reducing activity or having no activity compared to the endogenous activity. The weakening may be interchangeably used with terms such as inactivation, deficiency, down-regulation, decrease, reduce, attenuation, and the like.

The weakening may also include cases where the activity of the polypeptide itself is reduced or removed compared to activity of the polypeptide possessed by the original microorganism due to mutation of a polynucleotide encoding the polypeptide and the like, cases where the overall polypeptide activity level and/or concentration (expression level) is lower than a natural strain in cells due to inhibition of expression or inhibition of translation into a polypeptide of a gene of a polynucleotide encoding the same, and the like, cases where expression of the polynucleotide is not done at all, and/or cases where there is no activity of the polypeptide even if the polynucleotide is expressed. The "endogenous activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide is "inactivated, deficient, decreased, down-regulated, reduced, or attenuated" compared to the endogenous activity, refers to that it is lowered compared to the activity of a specific polypeptide originally possessed by a parent strain before trait changes or non-modified microorganism.

This weakening of the activity of the polypeptide may be performed by any method known in the art, but not limited thereto, and may be achieved by application of various methods well known in the art (for example, Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide may be

1) deficiency of the entire or a part of a gene encoding a polypeptide;

2) modification of an expression regulatory region (or expression regulatory sequence) so that expression of a gene encoding a polypeptide is reduced;

3) modification (for example, deletion/substitution/addition of at least one amino acid in the amino acid sequence) of an amino acid sequence composing the polypeptide so that the activity of the polypeptide is removed or weakened;

4) modification (for example, addition/substitution/addition of at least one nucleic acid base in the nucleic acid base sequence of the polypeptide gene to encode a polypeptide modified so that the activity of the polypeptide is removed or weakened) of a gene sequence encoding the polypeptide so that the activity of the polypeptide is removed or weakened;

5) modification of a base sequence encoding an initiation codon or 5'-UTR area of gene transcriptome encoding a polypeptide;

6) introduction of an antisense oligonucleotide (for example, antisense RNA) complementarily binding to transcriptome of a gene encoding a polypeptide;

7) addition of a sequence complementary to Shine-Dalgarno sequence in front of the Shine-Dalgarno sequence of a gene encoding a polypeptide so as to form a secondary structure incapable of attachment of ribosome;

8) addition of a promoter transcribed in the opposite direction at the 3' end of an ORF (open reading frame) of a gene sequence encoding a polypeptide (Reverse transcription engineering, RTE); or

9) adjustment of cellular localization of a protein (polypeptide); or

10) a combination of at least 2 selected from the 1) to 9), but not particularly limited thereto.

For example,
the 1) deficiency of a part or the entire of a gene encoding a polypeptide may be removal of the entire polynucleotide encoding an endogenous target polypeptide in chromosome, replacement with a polynucleotide in which some nucleotides are deleted, or replacement with a marker gene.

In addition, the 2) modification of an expression regulatory region (or expression regulatory sequence) may be mutation occurrence on the expression regulatory region (or expression regulatory sequence) by deletion, insertion, non-conservative or conservative substitution or a combination thereof, or replacement with a sequence having weaker activity. The expression regulatory region includes a promoter, an operator sequence, a sequence encoding a ribosome binding site, and a sequence regulating termination of transcription and translation, but not limited thereto.

Furthermore, the 3) modification of a base sequence encoding an initiation codon or 5'-UTR area of gene transcriptome encoding a polypeptide may be for example, substituting it with a base sequence encoding another initiation codon with a lower polypeptide expression rate compared to the endogenous initiation codon, but not limited thereto.

In addition, the modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have weaker activity or an amino acid sequence or polynucleotide sequence improved to have no activity, but not limited thereto. For example, by forming a stop codon by introducing mutation in a polynucleotide sequence, expression of a gene may be inhibited or weakened, but not limited thereto.

The 6) introduction of an antisense oligonucleotide (for example, antisense RNA) complementarily binding to transcriptome of a gene encoding a polypeptide may refer to for example, the document [Weintraub, H. et al., Antisense-RNA as a molecular tool for genetic analysis, Reviews - Trends in Genetics, Vol. 1(1) 1986].

The 7) addition of a sequence complementary to Shine-Dalgarno sequence in front of the Shine-Dalgarno sequence of a gene encoding a polypeptide so as to form a secondary structure incapable of attachment of ribosome may make mRNA translation impossible or inhibit its speed.

The 8) addition of a promoter transcribed in the opposite direction at the 3' end of an ORF (open reading frame) of a gene sequence encoding a polypeptide (Reverse transcription engineering, RTE) may create an antisense nucleotide complementary to the transcriptome of the gene encoding the polypeptide to weaken the activity.

The 9) adjustment of cellular localization of a polypeptide may target a polypeptide to an intracellular specific organelle or specific intracellular space. For example, by addition or removal of a leader sequence functioning in targeting a polypeptide, it may target it to periplasm or cytoplasm, but not limited thereto.

Such weakening of the activity of the polypeptide, may weaken the activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the present disclosure, the term, "enhancement" of the polypeptide activity, means that the activity of the polypeptide is increased compared to the endogenous activity. The enhancement may be interchangeably used with terms such as activation, up-regulation, overexpression, increase and the like. Herein, the activation, up-regulation, overexpression, and increase may include showing activity which is not originally present, or showing activity improved compared to the endogenous activity or activity before modification. The "endogenous activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide "is enhanced", "is up-regulated", "is overexpressed" or "increases" compared to the endogenous activity, refers to that it is improved compared to the activity and/or concentration (expression level) of a specific polypeptide originally possessed by a parent strain before trait changes or non-modified microorganism.

The enhancement may be achieved by introducing a foreign polypeptide, or enhancement of activity of an endogenous polypeptide and/or concentration (expression level). Whether the activity of the polypeptide is enhanced may be confirmed by the degree of activity, expression level of the corresponding polypeptide, or an increase of the amount of products released from the corresponding polypeptide.

For the enhancement of the activity of the polypeptide, various methods well known in the art may be applied, and there is no limitation, as long as the activity of a target polypeptide can be enhanced compared to a microorganism before modification. Specifically, genetic engineering and/or protein engineering well known to those skilled in the art, which are routine methods of molecular biology, may be used, but not limited thereto (for example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be

1) increasing the intracellular copy number of a polynucleotide encoding the polypeptide;
2) replacing a gene expression regulatory region on a chromosome encoding the polypeptide with a sequence having strong activity;
3) modification of a nucleotide sequence encoding an initiation codon or 5'-UTR area of gene transcriptome encoding a polypeptide;
4) modification of an amino acid sequence of the polypeptide so that the activity of the polypeptide is enhanced;
5) modification of a polynucleotide sequence encoding the polypeptide such that the polypeptide activity is enhanced (for example, modification of a polynucleotide of the polypeptide gene so as to encode a polypeptide modified to enhance the activity of the polypeptide);
6) introduction of a foreign polypeptide exhibiting the activity of the polypeptide or a foreign polynucleotide encoding the same;
7) codon optimization of a polynucleotide encoding a polypeptide;
8) modification or chemical modification by analyzing the tertiary structure of a polypeptide and selecting an exposed site;
9) adjustment of cellular localization of a protein (polypeptide); or
10) a combination of at least 2 selected from the 1) to 9), but not particularly limited thereto.

More specifically,

The increase in the intracellular copy number of a polynucleotide encoding the polypeptide of 1) may be achieved by introduction of a vector which can replicate and function regardless of a host, in which a polynucleotide encoding the corresponding polypeptide is operably linked. Otherwise, it may be achieved by introduction of 1 copy or 2 copies or more of the polynucleotide encoding the corresponding polypeptide into chromosome in a host cell. The introduction into the chromosome may be performed by introducing a vector capable of inserting the polynucleotide into chromosome of the host cell into the host cell, but not limited thereto. The vector is as described above.

The 2) replacement of a gene expression regulatory region (or expression regulatory sequence) on chromosome encoding a polypeptide with a sequence having strong activity, may be for example, occurrence of mutation in the sequence by deletion, insertion, non-conservative or conservative substitution or a combination thereof so as to further intensify activity of the expression regulatory region, or replacement with a sequence having stronger activity. The expression regulatory region is not particularly limited thereto, but may comprise a promoter, an operator sequence, a sequence encoding a ribosome binding site, and a sequence regulating termination of transcription and translation and the like. As one example, the original promoter may be replaced with a strong promoter, but not limited thereto.

Examples of the known strong promoter include CJ1 to CJ7 promoters (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter, and the like, but not limited thereto.

The 3) modification of a base sequence encoding an initiation codon or 5'-UTR area of gene transcriptome encoding a polypeptide may be for example, substituting it with a base sequence encoding another initiation codon with a higher polypeptide expression rate compared to the endogenous initiation codon, but not limited thereto.

The modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have stronger activity for the amino acid sequence of the polypeptide or a polynucleotide sequence encoding the polypeptide so as to strengthen the activity of the polypeptide, but not limited thereto. The replacement may be performed specifically by inserting a polynucleotide into chromosome by homologous recombination, but not limited thereto. The vector used then may further comprise a selection marker for confirming chromosome insertion. The selection marker is as described above.

The 6) introduction of a foreign polypeptide showing activity of a polypeptide may be introduction of a foreign polynucleotide encoding a polypeptide showing the same/similar activity to the polypeptide into a host cell. The foreign polynucleotide is not limited in its origin or sequence as long as it shows the same/similar activity to the polypeptide. The method used for the introduction may be performed by those skilled in the art by appropriately selecting a known transformation method, and by expressing the introduced polynucleotide in a host cell, a polypeptide may be produced and its activity may be increased.

The 7) codon optimization of a polynucleotide encoding a polypeptide may be codon optimization so that transcription or translation of an endogenous polynucleotide is increased in a host cell, or codon optimization so that a foreign polynucleotide is optimized in a host cell to achieve transcription and translation.

The selecting, modifying, or chemically modifying an exposed site by analysis of the tertiary structure of the polypeptide of 8) above may be, for example, determining a template protein candidate according to the degree of sequence similarity by comparing the sequence information of the polypeptide to be analyzed with a database in which sequence information of known proteins is stored, and identifying the structure based thereon, then selecting, modifying, or modifying an exposed site to be modified or chemically modified.

The 9) adjustment of cellular localization of a polypeptide may target a polypeptide into an intracellular specific organelle or specific intracellular space. For example, by addition or removal of a leader sequence functioning in targeting a polypeptide, it may target it to periplasm or cytoplasm, but not limited thereto.

Such enhancement of the polypeptide activity means increasing activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the microorganism of the present disclosure, modification of a part or all of a polynucleotide (for example, modification to encode the afore-mentioned protein variant) may be induced by (a) homologous recombination using a vector for inserting chromosome into a microorganism or genome editing using genetic scissors (engineered nuclease, e.g., CRISPR-Cas9) and/or (b) treatment of light such as ultraviolet rays and radioactive rays and the like and/or chemical substances, but not limited thereto. The method of modifying a part of all of the gene may include a method for DNA recombination technology. For example, by injecting a nucleotide sequence or vector comprising a nucleotide sequence homologous to a target gene into the microorganism to cause homologous recombination, deletion of a part or all of the gene may be achieved. The injected nucleotide sequence or vector may include a dominant selection marker, but is not limited thereto.

In the microorganism of the present disclosure, the variant, polynucleotide and glutamate family amino acids and the like are as described in other aspects above.

Another aspect of the present disclosure provides a method for producing glutamate family amino acids, comprising culturing a microorganism of the genus *Corynebacterium* comprising the variant of the present disclosure or the polynucleotide of the present disclosure.

The method of producing glutamate family amino acids of the present disclosure may comprise culturing a microorganism of the genus *Corynebacterium* comprising the variant of the present disclosure or the polynucleotide of the present disclosure in a medium.

In addition, the glutamate family amino acids of the present disclosure may be at least one selected from the group consisting of L-ornithine, L-citrulline, L-arginine and putrescine.

In the present disclosure, "culturing" means growing a microorganism of the genus *Corynebacterium* of the present disclosure under an appropriately adjusted environmental condition. The culturing process of the present disclosure may be conducted according to an appropriate medium and culturing conditions known in the art. This culturing process may be easily adjusted and used by those skilled in the art depending on the selected strain. Specifically, the culturing may be batch, continuous, and/or fed-batch, but not limited thereto.

In the present disclosure, the term "medium" refers to a material in which nutrients required for culturing the microorganism of the genus *Corynebacterium* are mixed as main components, and supplies nutrients and growth factors and the like including water which is essential for survival and growth. Specifically, the medium used for culturing the microorganism of the genus *Corynebacterium* of the present disclosure and other culturing conditions may be used as long as it is a medium used for culturing a common microorganism without particular limitation, but the microorganism of the genus *Corynebacterium* of the present disclosure may be cultured in a common medium containing a suitable carbon source, a nitrogen source, a phosphorus source, an inorganic compound, an amino acid and/or a vitamin and the like by adjusting temperature, pH and the like under an aerobic condition.

Specifically, the culture medium for the microorganism of the genus *Corynebacterium* may be found in the document ["Manual of Methods for General Bacteriology" by the American Society for Bacteriology (Washington D.C., USA, 1981)].

In the present disclosure, as the carbon source, carbohydrates such as glucose, saccharose, lactose, fructose, sucrose, maltose, etc.; sugar alcohols such as mannitol, sorbitol, etc., organic acids such as pyruvate, lactate, citrate, etc.; amino acids such as glutamic acid, methionine, lysine, etc. and the like, may be included. In addition, natural organic nutrients such as starch hydrates, molasses, blackstrap molasses, rice bran, cassava, sugar cane residues, and corn steep liquor may be used, and specifically, carbohydrates such as glucose and sterilized pre-treated molasses (that is, molasses converted with reducing sugars) and the like may be used, and other carbon sources in an appropriate amount may be variously used without limitation. These carbon sources may be used alone or at least two kinds may be used in combination, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like; and organic nitrogen sources such as amino acids including glutamic acid, methionine, glutamine and the like, peptone, NZ-amine, meat extract, yeast extract, malt extract, corn steep liquor, casein hydrates, fish or degraded products thereof, defatted soybean cake or degraded products thereof, and the like may be used. These nitrogen sources may be used alone or at least two may be used in combination, but not limited thereto.

As the phosphorus source, potassium phosphate monobasic, potassium phosphate dibasic, or sodium-containing salts corresponding thereto, or the like may be included. As the inorganic compound, sodium chloride, calcium chloride, iron chloride, magnesium sulfate, iron sulfate, manganese sulfate, calcium carbonate and the like may be used, and in addition thereto, amino acids, vitamins, and/or appropriate precursors and the like may be included. These components or precursors may be added in a batch or continuous method. However, it is not limited thereto.

In addition, by adding a compound such as ammonium hydroxide, potassium hydroxide, ammonia, phosphoric acid, sulfuric acid and the like to a medium during culturing of the microorganism of the genus *Corynebacterium* of the present disclosure by an appropriate method, the pH of the medium may be adjusted. Furthermore, during culturing, foam generation may be inhibited using an antifoaming agent such as fatty acid polyglycol ester. Moreover, in order to maintain the aerobic state of the medium, oxygen or oxygen-containing gas may be injected into the medium, or in order to maintain the anaerobic and microaerobic state, gas may be not injected or nitrogen, hydrogen or carbon dioxide gas may be injected, but not limited thereto.

In the culturing of the present disclosure, the culturing temperature may be maintained at 20 to 45°C, specifically, 25 to 40°C, and the culturing may be carried out for about 10 to 160 hours, but is not limited thereto.

The glutamate family amino acids produced by the culturing of the present disclosure may be released to a medium or remain in cells.

The method of producing glutamate family amino acids of the present disclosure, may further comprise preparing the microorganism of the genus *Corynebacterium* of the present disclosure, preparing a medium for culturing the strain, or a combination thereof (in any order), for example, before the culturing.

The method of producing glutamate family amino acids of the present disclosure, may further comprise recovering glutamate family amino acids from the medium according to the culturing (medium in which culturing is performed) or the microorganism of the genus *Corynebacterium*. The recovering may be further comprised after the culturing.

The recovering may collect targeted glutamate family amino acids using an appropriate method known in the art according to the culturing method of the microorganism of the present disclosure, for example, a batch, continuous or fed-batch culturing method, or the like. For example, centrifugation, filtration, treatment by a crystallized protein precipitator (salting out), extraction, ultrasonic crushing, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and using an appropriate method known in the art, targeted glutamate family amino acids may be recovered from a medium or microorganism.

In addition, the method of producing glutamate family amino acids of the present disclosure, may additionally comprise a purification step. The purification may be performed, using an appropriate method known in the art. In one embodiment, when the method of producing glutamate family amino acids of the present disclosure comprises both the recovering step and purification step, the recovering step and purification step may be continuously or non-continuously performed regardless of the order, or may be performed by being integrated as one step, but not limited thereto.

In the method of the present disclosure, the variant, polynucleotide, vector and strain and the like are described in other aspects above.

Another aspect of the present disclosure provides a composition for producing glutamate family amino acids comprising the variant of the present disclosure, a polynucleotide encoding the variant, a vector comprising the polynucleotide, or a microorganism of the genus *Corynebacterium* comprising the polynucleotide of the present disclosure; a medium culturing the same; or a combination of at least 2 among them.

The composition of the present disclosure may further comprise any appropriate excipient commonly used for compositions for producing amino acids, and such an excipient, may be for example, a preservative, wetting agent, dispersant, suspending agent, buffer, stabilizer, or tonicifying agent, or the like, but not limited thereto.

In the composition of the present disclosure, the variant, polynucleotide, vector, microorganism, medium and glutamate family amino acids and the like are as described in other aspects above.

Another aspect of the present disclosure provides a use for producing glutamate family amino acids of a microorganism of the genus *Corynebacterium* comprising the variant of the present disclosure, a polynucleotide encoding the variant, a vector comprising the polynucleotide, or a microorganism of the genus *Corynebacterium* comprising the polynucleotide of the present disclosure.

The variant, polynucleotide, vector, microorganism, glutamate family amino acids, and the like are as described in the other aspects above.

Another aspect of the present disclosure provides a use for the preparation of a composition for producing glutamate family amino acids of a microorganism of the genus *Corynebacterium* comprising the variant of the present disclosure, a polynucleotide encoding the variant, a vector comprising the polynucleotide, or a microorganism of the genus *Corynebacterium* comprising the polynucleotide of the present disclosure.

The variant, polynucleotide, vector, microorganism, glutamate family amino acids, and the like are as described in the other aspects above.

According to other aspects of the present disclosure, the present disclosure provides a composition, method, product, process, or use characterized by one or more elements disclosed in the present disclosure.

[ADVANTAGEOUS EFFECTS]

When a microorganism of the genus *Corynebacterium* comprising the protein variant of the present disclosure is cultured, it is possible to produce glutamate family amino acids at a higher yield as compared to a microorganism having an existing unmodified polypeptide.

[MODE FOR INVENTION]

Hereinafter, the present disclosure will be described in more detail by Examples. However, the following Examples are merely preferred embodiments for illustrating the present disclosure, and therefore, are not intended to limit the scope of rights of the present disclosure thereto. Meanwhile, technical matters not described in the present disclosure may be sufficiently understood and readily carried out by those skilled in the art to which the present disclosure pertains or in a similar technical field.

**Example 1: Construction of vectors expressing wild-type bifunctional glutamate N-acetyltransferase/amino acid acetyltransferase and variants thereof**

In order to confirm the effect of a variant (I201F+D355E; SEQ ID NO: 1), in which isoleucine (Ile) at position 201 is substituted with phenylalanine (Phe) and aspartic acid (Asp) at position 355 is substituted with glutamic acid (Glu), of the dual-functional glutamate N-acetyltransferase/amino acid acetyltransferase amino acid sequence (SEQ ID NO: 3), on the production of glutamate family amino acids, vectors capable of expressing the *argJ* gene encoding wild-type and mutant (I201F+D355E) bifunctional glutamate N-acetyltransferase/amino acid acetyltransferase were constructed as follows.

Using the genome (NZ_CP016335.1) of wild-type Corynebacterium glutamicum ATCC13869 as a template, PCR was performed using a primer pair of SEQ ID NOs: 5 and 10 to amplify gene fragment A (1167 bp). At this time, the PCR reaction was carried out by denaturation at 95 °C for 10 minutes, followed by 30 cycles of denaturation at 95 °C for 30 seconds, annealing at 55 °C for 30 seconds, and polymerization at 72 °C for 1 minute, and then a polymerization reaction at 72 °C for 5 minutes. Thereafter, the PCR fragments were obtained using a gel purification kit (QIAGEN). In order to amplify gene fragments B (609 bp), C (483 bp), and D (126 bp) for introducing the mutation (I201F+D355E), PCR was performed by the above method using a primer pair of SEQ ID NOs: 5 and 6, a primer pair of SEQ ID NOs: 7 and 8, and a primer pair of SEQ ID NO: 9 and SEQ ID NO: 10, respectively, to obtain gene fragments B, C, and D. In addition, in order to secure expression promoters in the vectors, PCR was performed by the above method using a primer pair of SEQ ID NOs: 11 and 12, using a gene upstream of *NCgl0856* of wild-type *Corynebacterium glutamicum* ATCC13869 as a template, thereby obtaining a promoter region gene fragment of 500 bp.

The promoter region gene fragment obtained above and gene fragment A, and the promoter region gene fragment and gene fragments B, C, and D were fusion-cloned into a pCES208 vector (SEQ ID NO: 50) cleaved with the restriction enzymes of BamHI and XbaI, using an In-Fusion Cloning Kit.

The plasmid prepared above was transformed into *Escherichia coli* DH5α and spread onto LB solid medium containing kanamycin (25 mg/L). From the selected colonies, plasmids were obtained using a conventionally known plasmid extraction method, and the vector containing gene fragment A and the promoter was named "pCES208-PbetP-*argJ*", and the vector containing gene fragments B, C, and D and the promoter was named "pCES208-PbetP-*argJ*(I201F, D355E)".

The primer sequences used in Example 1 are shown in Table 1 below.

[Table 1]

| Name | Sequence (5'->3') | SEQ ID NO: |
|---|---|---|
| Primer 1 | CAAATTCGAAACCGATAATGGCCAAAAAAGGCAT | SEQ ID NO: 5 |
| Primer 2 | CAGCGCAAaCTGAGCCATTTCCTGAGTAACGGAT | SEQ ID NO: 6 |
| Primer 3 | TGGCTCAGtTTGCGCTGGCTAATGCTACGGCCGT | SEQ ID NO: 7 |
| Primer 4 | CAATTCGGACcTCAATGTCAGCGCCGGAAAGATC | SEQ ID NO: 8 |
| Primer 5 | CATTGAgGTCCGAATTGATTTGGGCACCAGTGGG | SEQ ID NO: 9 |
| Primer 6 | GTGGCGGCCGCTCTAGATTAAGAGCTGTACGCGG | SEQ ID NO: 10 |
| PbetP_F | TGCAGCCCGGGGGATCCCAGCCGAAGTTTTAGGT | SEQ ID NO: 11 |
| PbetP_R | TATCGGTTTCGAATTTGGGTCAGATGTAGTCATA | SEQ ID NO: 12 |

**Example 2. Evaluation of production ability of L-ornithine of microorganisms expressing bifunctional glutamate N-acetyltransferase/amino acid acetyltransferase variants**

**2-1. Preparation of microorganisms producing L-ornithine**

In order to prepare L-ornithine-producing microorganisms, a vector in which glutamic acid located at position 47 of the amino acid sequence of ArgR (ANU33619.1) was substituted with a stop codon was prepared.

Using the genome of wild-type *Corynebacterium glutamicum* ATCC13869 as a template, a homologous recombinant A arm was amplified using a primer pair of SEQ ID NOs: 13 and 14, and a homologous recombinant B arm was amplified using a primer pair of SEQ ID NOs: 15 and 16. At this time, the PCR conditions were such that denaturation was performed at 95 °C for 10 minutes, followed by 30 cycles of denaturation at 95 °C for 30 seconds, annealing at 55 °C for 30 seconds, and polymerization at 72 °C for 1 minute, and then a polymerization at 72 °C for 5 minutes. The amplified homologous recombinant arms and a vector pDC24 (SEQ ID NO: 51) cleaved with the restriction enzymes of BamHI and XbaI were cloned to obtain a plasmid. This plasmid was named pDC24-*argR*(E47*).

In order to prepare a microorganism having further improved L-ornithine-production ability, a vector in which serine located at position 55 of the protein sequence of ArgF (ANU33618.1) was substituted with a stop codon was prepared. Using the genome of *Corynebacterium glutamicum* ATCC13869 as a template, a homologous recombinant C arm was amplified using a primer pair of SEQ ID NOs: 17 and 18, and a homologous recombinant D arm was amplified using a primer pair of SEQ ID NOs: 19 and 20. Thereafter, a plasmid was obtained by the same method as described above, and this plasmid was named pDC24-*argF*(S55*).

Using the constructed pDC24-argR(E47*) vector, wild-type *Corynebacterium glutamicum* ATCC13869 was transformed by electroporation (Appl. Microbiol. Biotechnol. (1999) 52:541-545), and after a second crossover process, a microorganism was prepared in which the 139th nucleotide sequence of *argR* was substituted from guanine (G) to thymine (T), thereby substituting the 47th protein sequence with a stop codon. PCR and sequencing were performed using a primer pair of SEQ ID NOs: 13 and 16 capable of amplifying an adjacent region including the site into which the gene was inserted, and the corresponding genetic manipulation was confirmed. The microorganism thus obtained was named C. *gl::argR*.

Using the pDC24-argF(S55*) vector in C. gl::*argR** a microorganism was prepared in which the 164th nucleotide sequence of *argF* as substituted from cytosine (C) to adenine (A), thereby substituting the 55th protein sequence with a stop codon. PCR and sequencing were performed using a primer pair of SEQ ID NOs: 17 and 20 capable of amplifying an adjacent region including the site into which the gene was inserted, and the corresponding genetic manipulation was confirmed. The microorganism thus obtained was named C. gl::*argR*_argF**.

The primer sequences used in Example 2 are shown in Table 2 below.

[Table 2]

| Name | Sequence (5'->3') | SEQ ID NO: |
|---|---|---|
| Primer 7 | CGGTACCCGGGGATCCCTCGTGCGGAATTCGTGGAG | SEQ ID NO: 13 |
| Primer 8 | ATCCAGCAGCAATTCAGACA | SEQ ID NO: 14 |
| Primer 9 | CTGAATTGCTGCTGGATTAAGGCATCGATATCACCCA | SEQ ID NO: 15 |
| Primer 10 | ATGCCTGCAGGTCGACCCTTCATTTTAAGTTCCTTG | SEQ ID NO: 16 |
| Primer 11 | CGGTACCCGGGGATCCTGACCCCAGGCAAGCACGG | SEQ ID NO: 17 |
| Primer 12 | GAAGCGAGTACGAGTTTAAGTCTTATC | SEQ ID NO: 18 |
| Primer 13 | AAACTCGTACTCGCTTCTCC | SEQ ID NO: 19 |
| Primer 14 | ATGCCTGCAGGTCGACCGGCGCCGGCAACCTCGTC | SEQ ID NO: 20 |

## 2-2. Confirmation of increased production ability of L-ornithine of microorganisms expressing bifunctional glutamate N-acetyltransferase/amino acid acetyltransferase variants

The vectors prepared in Example 1 were transformed into the C. gl::*argR*_argF** strain prepared in Example 2-1 by electroporation, thereby obtaining strains into which the vectors were introduced.

Specifically, the strain into which pCES208-PbetP-*argJ* was introduced was named "C. gl::*argR*_argF*-argJ*", and the strain into which pCES208-PbetP-*argJ*(I201F, D355E) was introduced was named "C. gl::*argR*_argF*-argJ* (I201F, D355E)".

In order to analyze L-ornithine production ability, a control strain, C. gl::*argR*_argF*-argJ* strain, and C. gl:: *argR*_argF*-argJ*(I201F, D355E) strain were cultured by the method described below, and $OD_{562}$, L-ornithine production amount and L-ornithine production yield were measured.

Specifically, each strain was inoculated into a 250 mL corner-baffled flask containing 25 mL of the production medium described below, and cultured with shaking at 200 rpm at 33 °C for 48 hours. After completion of the culture, the culture broth was diluted 100-fold with a 0.1 N HCl solution, and $OD_{562}$ was measured using a spectrophotometer. The production amount of L-ornithine and the L-ornithine production yield were measured using HPLC. The yield was calculated as a ratio (%) obtained by dividing the production amount by the consumed sugar, as shown in Equation 1 below.

[Equation 1]

$$\text{Yield(\%)} = (\text{Production amount of product(g/L)/Consumed sugar(g/L)}) \times 100$$

<Production medium (pH 7.2)>

Raw sugar 50 g, $(NH_4)_2SO_4$ 40 g, yeast extract 1 g, $KH_2PO_4$ 1.1 g, $MgSO_4 \cdot 7H_2O$ 1.2 g, L-arginine 0.2 g, biotin 1 mg, thiamine hydrochloride 5 mg, calcium-pantothenate 5 mg, nicotinamide 15 mg, $MnSO_4$ 10 mg, $FeSO_4$ 10 mg, $ZnSO_4$ 0.5 mg, $CuSO_4$ 0.5 mg, $CaCO_3$ 30 g, kanamycin 25mg (based on 1 liter of distilled water)

The above experiment was repeated three times, and the average values of the analysis results are shown in Table 3 below.

[Table 3]

| Strain name | OD$_{562}$ | Consumed sugar (g/L) | L-ornithine production amount (g/L) | L-ornithine yield (%) |
|---|---|---|---|---|
| C. g1::argR*_argF*-argJ | 38.0 | 50.0 | 16.2 | 32.5 |
| C. g1::argR*_argF*-argJ(I201F + D355E) | 36.9 | 50.0 | 19.0 | 38.0 |

As a result, as shown in Table 3 above, it was confirmed that the L-ornithine production ability was increased by an average of 17% in the C. gl::argR*_argF*-argJ(I201F + D355E) strain into which the mutant argJ(I201F + D355E) was introduced, as compared to the C. gl::argR*_argF*-argJ strain into which the wild-type argJ was introduced.

From the above results, it was confirmed that the bifunctional glutamate N-acetyltransferase/amino acid acetyltransferase variant ArgJ(I201F + D355E) selected in the present disclosure increased the L-ornithine production ability of Corynebacterium glutamicum.

**Example 3. Evaluation of L-citrulline production ability of microorganisms expressing bifunctional glutamate N-acetyltransferase/amino acid acetyltransferase variants**

**3-1. Preparation of an L-citrulline-producing microorganism**

In order to prepare an L-citrulline-producing microorganism, a vector in which phenylalanine located at position 68 of the protein sequence of ArgG (ANU33620.1) was substituted with a stop codon was prepared.

Using the genome of wild-type Corynebacterium glutamicum ATCC13869 as a template, a homologous recombinant A arm was amplified using a primer pair of SEQ ID NOs: 21 and 22, and a homologous recombinant B arm was amplified using a primer pair of SEQ ID NOs: 23 and 24. Thereafter, a plasmid was obtained by the same method as described in Example 2-1, and this plasmid was named pDC24-argG(F68*).

In order to prepare a microorganism having further improved L-citrulline production ability in C. gl::argR* prepared in Example 2-1, a microorganism was prepared using the pDC24-argG(F68*) vector, in which the 203rd nucleotide sequence of argG was substituted from thymine (T) to adenine (adenine, A), and the 204th nucleotide sequence was substituted from cytosine (cytosine, C) to adenine (adenine, T), thereby substituting the 68th protein sequence with a stop codon. PCR and sequencing were performed using a primer pair of SEQ ID NOs: 21 and 24 capable of amplifying an adjacent region including the site into which the gene was inserted, and the corresponding genetic manipulation was confirmed. The microorganism thus obtained was named C. gl::argR*_argG*.

The primer sequeces used in Example 3 are shown in Table 4 below.

[Table 4]

| Name | Sequence (5'->3') | SEQ ID NO: |
|---|---|---|
| Primer 15 | CGGTACCCGGGGATCCTTCATCGATAGGGTGGG | SEQ ID NO: 21 |
| Primer 16 | GTACTCCTCAGCTTACTCATCCTTTGCATCAACA | SEQ ID NO: 22 |
| Primer 17 | AGTAAGCTGAGGAGTACTGCCTGCCAACCATCA A | SEQ ID NO: 23 |

(continued)

| Name | Sequence (5'->3') | SEQ ID NO: |
|---|---|---|
| Primer 18 | ATGCCTGCAGGTCGACCGACTGGCTTGCCACCCT | SEQ ID NO: 24 |

**Example 3-2: Confirmation of increased L-citrulline production ability of microorganisms expressing bifunctional glutamate N-acetyltransferase/amino acid acetyltransferase variants**

The vectors prepared in Example 1 were transformed into the C. gl::*argR*_argG** strain prepared in Example 3-1 by electroporation, thereby obtaining strains into which the vectors were introduced.

Specifically, the strain into which pCES208-PbetP-*argJ* was introduced was named "C. gl::*argR*_argG*-argJ*", and the strain into which pCES208-PbetP-*argJ*(I201F + D355E) was introduced was named "C. gl::*argR*_argG*-argJ* (I201F + D355E)".

In order to analyze L-citrulline production ability, a control strain, *Corynebacterium glutamicum* C. gl::*argR*_argG*-argJ* strain, and *Corynebacterium glutamicum* C. gl::*argR*_argG*-argJ*(I201F + D355E) strain were cultured by the method described below, and $OD_{562}$, L-citrulline production amount and L-citrulline production yield were measured.

Specifically, each strain was inoculated into a 250 mL corner-baffled flask containing 25 mL of the production medium described below, and cultured with shaking at 200 rpm at 33 °C for 48 hours. After completion of the culture, $OD_{562}$ of the culture broth was measured, and the amount of L-citrulline produced and L-citrulline yield were measured using HPLC.

<Production medium (pH 7.2)>

Raw sugar 50 g, $(NH_4)_2SO_4$ 40 g, yeast extract 1 g, $KH_2PO_4$ 1.1 g, $MgSO_4·7H_2O$ 1.2 g, L-arginine 0.2 g, biotin 1 mg, thiamine hydrochloride 5 mg, calcium-pantothenate 5 mg, nicotiamide 15 mg, $MnSO_4$ 10 mg, $FeSO_4$ 10 mg, $ZnSO_4$ 0.5 mg, $CuSO_4$ 0.5 mg, $CaCO_3$ 30 g, kanamycin 25mg (based on 1 liter of distilled water)

The above experiment was repeated three times, and the average values of the analysis results are shown in Table 5 below.

[Table 5]

| Strain name | $OD_{562}$ | Consumed sugar (g/L) | L-citrulline production amount (g/L) | L-citrulline yield (%) |
|---|---|---|---|---|
| C. gl::*argR*_argG*-argJ* | 44.4 | 50.0 | 4.8 | 9.6 |
| C. gl::*argR*_argG*-argJ*(I201F + D355E) | 46.0 | 50.0 | 6.1 | 12.2 |

As a result, as shown in Table 5 above, it was confirmed that the L-citrulline production ability was increased by an average of 27% in the C. gl::*argR*_argG*-argJ*(I201F + D355E) strain into which the mutant *argJ(I201F* + D355E) was introduced, as compared to the C. gl::*argR*_argG*-argJ* strain into which the wild-type *argJ* was introduced.

From the above results, it was confirmed that the bifunctional glutamate N-acetyltransferase/amino acid acetyltransferase variant ArgJ(I201F + D355E) selected in the present disclosure increased the L-citrulline production ability of *Corynebacterium glutamicum.*

**Example 4. Evaluation of L-arginine, L-ornithine and L-citrulline production ability of microorganisms producing L-arginine which express bifunctional glutamate N-acetyltransferase/amino acid acetyltransferase variants**

**Example 4-1. Preparation of microorganisms producing L-arginine**

*Corynebacterium glutamicum* CJR2 strain, in which the *argR* gene was deleted and an *argB*(M54V) gene mutation was introduced, and *Corynebacterium glutamicum* CJR100 strain, in which the *argR* gene was deleted and an *argB*(M54V) gene mutation and *argC* gene enhancement were introduced, were prepared in *Corynebacterium glutamicum* ATCC13869 strain as described below.

First, in order to prepare the *Corynebacterium glutamicum* CJR2 strain, vectors for *argR* deletion and introduction of an *argB*(M54V) mutation were prepared as follows. Using genomic DNA of *Corynebacterium glutamicum* ATCC13869 as a template, PCR was performed using primer pairs of SEQ ID NOs: 25 and 26 and SEQ ID NOs: 27 and 28, and overlapping PCR was performed using a primer pair of SEQ ID NOs: 25 and 28, thereby obtaining a homologous recombination fragment having an *argR* deletion mutation sequence. In the same manner as described above, in order to prepare a homologous recombination fragment having an *argB*(M54V) mutation, PCR was performed using primer pairs of SEQ ID NOs: 29 and 30 and SEQ ID NOs: 31 and 32, and overlapping PCR was performed using a primer pair of SEQ ID NOs: 29 and 32. The PCR reaction was carried out by repeating 30 cycles of denaturation at 95°C for 30 seconds; annealing at 55°C for 30 seconds; and extension at 72°C for 2 minutes. Thereafter, the linearized pDC24 vector (SEQ ID NO: 51) and each homologous recombination fragment were fusion-cloned in the same manner as described in Example 2. The prepared vectors (recombinant plasmids) were named pDC24-Δ*argR* and pDC24-*argB*(M54V), respectively.

In order to introduce an *argR* deletion mutation into wild-type *Corynebacterium glutamicum* ATCC13869, the previously prepared pDC24-Δ*argR* plasmid was used to transform the strain by electroporation. Thereafter, PCR was performed on transformants in which a second recombination had been completed, using a primer pair of SEQ ID NOs: 25 and 28, to confirm that a deletion mutation had been introduced into the *argR* gene on the chromosome. At this time, the PCR reaction was carried out by repeating 30 cycles of denaturation at 95°C for 30 seconds; annealing at 55°C for 30 seconds; and extension at 72°C for 2 minutes. The transformant was named CJR1.

The *argB*(M54V) mutation was introduced into the *Corynebacterium glutamicum* CJR1 strain in the same manner as described above. The previously prepared pDC24-*argB*(M54V) plasmid was used, and PCR was performed on transformants in which second recombination had been completed, using a primer pair (SEQ ID NOs: 29 and 32), to confirm that the M54V mutation had been introduced into the *argB* gene on the chromosome, and the transformant was named *Corynebacterium glutamicum* CJR2 strain.

Using the prepared *Corynebacterium glutamicum* CJR2 strain, a CJR100 strain in which the N-acetyl-gamma-glutamyl-phosphate reductase gene (hereinafter, *argC*) was additionally enhanced, thereby improving L-arginine productivity, was prepared.

Specifically, in order to enhance the activity of the N-acetyl-gamma-glutamyl-phosphate reductase gene, *argC* (NCBI Accession No. BBD29_RS07535), a plasmid for enhancing *argC* activity was prepared by replacing the wild-type promoter of the *argC* gene with the o2 promoter, which is known as a strong promoter (Korean Registered Patent No. 10-1632642, hereinafter Po2). Specifically, in order to prepare a strain into which *argC* having Po2 was introduced, PCR was performed to amplify a gene fragment of the upstream region of the *argC* gene using primers of SEQ ID NOs: 33 and 34, and to amplify a gene fragment of the downstream region of the *argC* gene using primers of SEQ ID NOs: 35 and 36, with chromosomal DNA of *Corynebacterium glutamicum* ATCC13869 used as a template. In addition, an o2 promoter fragment was obtained by performing PCR using a primer pair of SEQ ID NOs: 37 and 38, with Po2 (SEQ ID NO: 39) used as a template.

As a polymerase for the PCR reaction, PfuUltraTM High-Fidelity DNA Polymerase (Stratagene) was used, and the PCR conditions were performed with denaturation at 95°C for 30 seconds; annealing at 55°C for 30 seconds; and extension at 72°C for 1 minute, and the denaturation, annealing, and extension reactions under the above conditions were repeated 28 cycles. As a result, an 86 bp (SEQ ID NO: 39) DNA fragment of the o2 promoter region, a 610 bp DNA fragment of the upstream region of *argC* of *Corynebacterium glutamicum* ATCC13869, and a 1086 bp DNA fragment of the downstream region were obtained, respectively. After the PCR fragments obtained above were subjected to DNA purification, the fragments were ligated to a pDC24 plasmid treated with the SmaI restriction enzyme using an In-Fusion® HD Cloning Kit (Clontech) for fusion cloning. The vector obtained as a result was named pDC24-Po2-*argC*.

Thereafter, the prepared pDC24-Po2_*argC* plasmid was used to transform the *Corynebacterium glutamicum* CJR2 strain by an electropulse method. Subsequently, a second recombination was performed on a solid plate medium containing 4% glucose, and PCR was performed on transformants in which the second recombination had been completed using a primer pair of SEQ ID NOs: 36 and 37, to confirm that the argC gene on the chromosome had been enhanced with Po2. At this time, the PCR reaction was carried out by repeating 30 cycles of denaturation at 95°C for 30 seconds; annealing at 55°C for 30 seconds; and extension at 72°C for 2 minutes. The transformant was named *Corynebacterium glutamicum* CJR100 strain.

<Complex plate medium (pH 7.0)>

Glucose 10 g, peptone 10 g, beef extract 5 g, yeast extract 5 g, Brain Heart Infusion 18.5 g, NaCl 2.5 g, urea 2 g, sorbitol 91 g, agar 20g (based on 1 liter of distailled water)

The primer sequences used in Example 4-1 were shown in Table 6 below.

[Table 6]

| Name | Sequence (5'-> 3') | SEQ ID NO: |
|------|--------------------|-----------|
| argR-5'-F | tgaattcgagctcggtaccccactggtgaactccttgtcc | SEQ ID NO: 25 |
| argR-5'-R | ttgaactaggggcgctttaaaagttttccggtgttgacgg | SEQ ID NO: 26 |
| argR-3'-F | ccgtcaacaccggaaaactttttaaagcgcccctagttcaa | SEQ ID NO: 27 |
| argR-3'-R | gtcgactctagaggatcccccgttgaactgcttgccagcc | SEQ ID NO: 28 |
| argB-5'-F | tgaattcgagctcggtaccctgcggctcgcacggttgctc | SEQ ID NO: 29 |
| argB-5'-R | acggtgcgcaagaagaccacgtcggcagcaaaagcagcct | SEQ ID NO: 30 |
| argB-3'-F | ggctgcttttgctgccgacgtggtcttcttgcgcaccgtg | SEQ ID NO: 31 |
| argB-3'-R | gtcgactctagaggatccccctcttatcaggccaatcggt | SEQ ID NO: 32 |
| argC-5'-F | GTGAATTCGAGCTCGGTACCCGCCCCGAAAAGCCGTTAAAAG | SEQ ID NO: 33 |
| argC-5'-R | tgccaaaattcacgattattgCTCGAGTCTAGAGACGGGTTA | SEQ ID NO: 34 |
| argC-3'-F | ttattggaggagatcaaaacaATGACAATCAAGGTTGCAATC | SEQ ID NO: 35 |
| argC-3'-R | CAGGTCGGCGTCGCACCTTAAGGGGATCCTCTAGAGTCGACC | SEQ ID NO: 36 |
| Po2-F | CAATAATCGTGAATTTTGGCAGCAACAGAATTAT | SEQ ID NO: 37 |
| Po2-R | TGTTTTGATCTCCTCCAATAATCTATGCTTTTGC | SEQ ID NO: 38 |

**Example 4-2. Evaluation of L-arginine, L-ornithine, and L-citrulline production ability of microorganisms producing L-arginine which express bifunctional glutamate N-acetyltransferase/amino acid acetyltransferase variants**

The two types of vectors prepared in Example 1 were transformed by electroporation into *Corynebacterium glutamicum* CJR100, which is the L-arginine-producing strain prepared in Example 4-1, thereby preparing strains expressing argJ wild-type and mutant variants. The prepared strains were named CJR100-*argJ* and CJR100-*argJ* (I201F + D355E).

In order to compare the L-arginine, L-citrulline, and L-ornithine production ability of the strains, the strains were cultured by the method described below. Specifically, each strain was inoculated into a 250 mL corner-baffled flask containing 25 mL of the production medium described below, and cultured with shaking at 200 rpm at 30°C for 44 hours. The composition of the production medium used in this Example is as follows.

<Production medium (pH 7.2)>

Glucose 50 g, $(NH_4)_2SO_4$ 57 g, $MgSO_4 \cdot 7H_2O$ 2 g, beet molasses 5 g, calcium chloride 1 mg, cobalt chloride 1 mg, $KH_2PO_4$ 2 g, biotin 0.01 mg, thiamine-HCl 0.1 mg, calcium pantothenate 2 mg, nicotinamide 3 mg, $FeSO_4$ 10 mg, $MnSO_4$ 10 mg, $ZnSO_4$ 0.02 mg, $CuSO_4$ 0.5 mg, $CaCO_3$ 30 g, kanamycin 25 mg (based on 1 liter of distilled water)

After completion of the culture, the production ability (concentration) of L-arginine, L-citrulline, and L-ornithine was analyzed three times using high-performance liquid chromatography (HPLC) (Waters 2478), and the analyzed concentrations of L-arginine, L-citrulline, and L-ornithine are shown in Table 7 below.

[Table 7]

| Strain name | L-arginine concentration (g/L) | | L-citrulline concentration (g/L) | L-ornithine concentration (g/L) |
|-------------|----------------|------------------------------|--------------------|--------------------|
| | 3BT average | Degree of improvement (%) | 3BT average | 3BT average |
| Control group CJR100/pCES208 | 5.9 | - | 1.00 | 0.20 |

(continued)

| Strain name | L-arginine concentration (g/L) | | L-citrulline concentration (g/L) | L-ornithine concentration (g/L) |
|---|---|---|---|---|
| | 3BT average | Degree of improvement (%) | 3BT average | 3BT average |
| CJR100/pCES208-PbetP_argJ | 6.1 | - | 1.01 | 0.24 |
| CJR100/pCES208-PbetP_argJ(I201F + D355E) | 6.3 | 3.3 | 1.09 | 0.26 |

As a result, as shown in Table 7 above, the parent strain, *Corynebacterium glutamicum* CJR100, produced approximately 5.9 g/L of L-arginine, the strain into which wild-type *argJ* was introduced produced 6.1 g/L of L-arginine, and the strain into which the *argJ*(I201F + D355E) variant was introduced produced 6.3 g/L of L-arginine, thereby showing an L-arginine production ability improved by 3.3% as compared to the strain into which wild-type *argJ* was introduced.

In addition, as compared to the strain into which wild-type *argJ* was introduced, the strain into which the *argJ* (I201F + D355E) variant was introduced showed L-citrulline and L-ornithine production abilities improved by 8.5% and 7.9%, respectively.

From the above results, it was confirmed that the bifunctional glutamate N-acetyltransferase/amino acid acetyltransferase variant ArgJ(I201F + D355E) selected in the present invention not only more efficiently produced L-citrulline and L-ornithine but also more efficiently produced L-arginine, thereby increasing the L-arginine production ability of microorganisms of the genus *Corynebacterium.*

## Example 5. Evaluation of putrescine production ability of microorganisms expressing bifunctional glutamate N-acetyltransferase/amino acid acetyltransferase variants

### 5-1. Preparation of microorganisms producing putrescine

In order to prepare microorganisms producing putrescine, information on a gene encoding ornithine decarboxylase derived from *Lactobacillus* sp. 30A strain and surrounding nucleotide sequences was obtained from the National Institutes of Health (NIH) GenBank (SEQ ID NO: 40).

Gene fragments for preparing a vector were obtained by PCR using sequences obtained through gene synthesis based on the sequences secured above as templates.

As a polymerase, SolgTM Pfu-X DNA Polymerase was used, and the PCR amplification conditions were performed by denaturation at 95°C for 5 minutes, followed by 30 cycles of denaturation at 95°C for 30 seconds; annealing at 55°C for 30 seconds; and extension at 72°C for 2 minutes, and then an extension reaction was performed at 72°C for 5 minutes.

More specifically, in order to amplify the LODC gene derived from the *Lactobacillus* sp. 30A strain, primers of SEQ ID NOs: 42 and 43 were prepared, and as a result of performing PCR using SEQ ID NO: 41 as a template, a 2196 bp gene fragment was obtained.

In order to secure an expression promoter in a vector, PCR was performed in the same manner as described above using primers of SEQ ID NOs: 44 and 45, using wild-type *Corynebacterium glutamicum* ATCC13869 as a template, thereby obtaining a 318 bp promoter region gene fragment.

In order to delete *NCgl1469* (SEQ ID NO: 52), which is a gene encoding an enzyme that acetylates putrescine, a homologous recombinant A arm was amplified using a primer pair of SEQ ID NOs: 46 and 47, and a homologous recombinant B arm was amplified using a primer pair of SEQ ID NOs: 48 and 49, using the genome of wild-type *Corynebacterium glutamicum* ATCC13869 as a template. The PCR conditions were performed by denaturation at 95°C for 10 minutes, followed by repeating 30 cycles of denaturation at 95°C for 30 seconds, annealing at 55°C for 30 seconds, and extension at 72°C for 1 minute, and then an extension reaction was performed at 72°C for 5 minutes.

By ligating the amplified promoter region, gene fragments derived from *Lactobacillus* sp. 30A strain, and the homologous recombinant arms with the vector pDC24 (SEQ ID NO: 50) cleaved with BamHI and XbaI restriction enzymes using an In-Fusion Cloning Kit, a deletion vector was obtained. This plasmid was named pDC24-ΔNCgl1469::Pcj7_LODC.

The primer sequences used in Example 5 were shown in Table 8 below.

[Table 8]

| Name | Sequence (5'->3') | SEQ ID NO: |
|---|---|---|
| LODC_F | GAAAGGAAACACTCATGTCCTCTTCACTTAAAATTGC | SEQ ID NO: 42 |
| LODC_R | CTTAAATCGCCTTCAGGGTTAATTATTATACCGGTCGTC | SEQ ID NO: 43 |
| Pcj7_F | CGCTATTCTGGTATCCAGAAACATCCCAGCGC | SEQ ID NO: 44 |
| Pcj7_R | TTTTAAGTGAAGAGGACATGAGTGTTTCCTTTCGTTGG | SEQ ID NO: 45 |
| Primer 19 | AGCTCGGTACCCGGGGATCCCAGGAATACAGCTGTTC | SEQ ID NO: 46 |
| Primer 20 | CTGGGATGTTTCTGGATACCAGAATAGCGAAATG | SEQ ID NO: 47 |
| Primer 21 | GACGACCGGTATAATAATTAACCCTGAAGGCGATTTAAG | SEQ ID NO: 48 |
| Primer 22 | GCCTGCAGGTCGACTCTAGAAATGGCAGAGTTGG | SEQ ID NO: 49 |

## 5-2. Confirmation of increased putrescine production ability of microorganisms expressing bifunctional glutamate N-acetyltransferase/amino acid acetyltransferase variants

The vectors prepared in Example 1 were transformed by electroporation into the C. gl::*argR* _argF*-*ΔNCgl1469*::Pcj7_LODC strain prepared in Example 5-1, thereby obtaining strains into which the vectors were introduced.

Specifically, the strain into which pCES208-PbetP-*argJ* was introduced was named "C. gl::*argR* _argF*-*ΔNCgl1469*::Pcj7_LODC-*argJ*", and the strain into which pCES208-PbetP-*argJ*(I201F + D355E) was introduced was named "C. gl::*argR*_argF*-*ΔNCgl1469*::Pcj7_LODC-*argJ*(I201F, D355E)".

In order to analyze putrescine production ability, a control strain, C. gl::*argR* _argF*-*ΔNCgl1469*::Pcj7_LODC-*argJ* strain and C. gl::*argR*_argF*-*ΔNCgl1469*::Pcj7_LODC-*argJ*(I201F + D355E) strain were cultured by the method described below, and $OD_{562}$, the production amount of putrescine, and putrescine yield were measured.

Specifically, each strain was inoculated into a 300 mL comer-baffled flask containing 25 mL of the production medium described below, and cultured with shaking at 200 rpm at 33°C for 47 hours. After completion of the culture, the production amount of putrescine and putrescine production yield were measured using HPLC.

<Production medium (pH 7.2)>

Glucose 60 g, CM (cane molasses) solution 20 g, $(NH_4)_2SO_4$ 50 g, $KH_2PO_4$ 0.72 g, $MgSO_4 \cdot 7H_2O$ 1.13 g, $MgCl_2$ 0.4 g, $MnSO_4$ 0.18 g, $ZnSO_4$ 0.9 mg, $CuSO_4$ 0.9 mg, $FeSO_4$ 0.18 g, biotin 0.9 mg, thiamine hydrochloride 9 mg, calcium-pantothenate 9 mg, nicotinamide 60 mg, L-arginine 0.15 g, CSL (corn steep liquor) 8.12 g, $CaCO_3$ 50 g, kanamycin 25 mg (based on 1 liter of distilled water)

The above experiment was repeated three times, and the average values of the analysis results are shown in Table 9.

[Table 9]

| Strain name | $OD_{562}$ | Consumed sugar (g/L) | Putrescine production amount (g/L) | Putrescine yield (%) |
|---|---|---|---|---|
| C. gl::*argR*_argF*-*ΔNCgl1469*::Pcj7_LODC -*argJ* | 44.9 | 47.8 | 3.6 | 7.5 |

(continued)

| Strain name | OD$_{562}$ | Consumed sugar (g/L) | Putrescine production amount (g/L) | Putrescine yield (%) |
|---|---|---|---|---|
| C. gl::*argR\*_argF\**-Δ*NCgl1469*::Pcj7_LODC -*argJ*(I201F + D355E) | 32.6 | 60 | 11.2 | 18.8 |

As a result, as shown in Table 9 above, it was confirmed that putrescine production ability was increased, by an average of 2.1-fold based on the amount produced, and by an average of 1.5-fold based on production yield, in the C. gl::*argR\*_argF\**-Δ*NCgl1469*::Pcj7_LODC-*argJ*(I201F, D355E) strain into which the mutant *argJ(I201F* + D355E) was introduced, as compared to the C. gl::*argR\*_argF\**-Δ*NCgl1469*::Pcj7_LODC-*argJ* strain into which the wild-type *argJ* was introduced.

From the above results, it was confirmed that the bifunctional glutamate N-acetyltransferase/amino acid acetyltransferase variant ArgJ(I201F + D355E) selected in the present invention increased the putrescine production ability of microorganisms of the genus *Corynebacterium*.

From the above description, those skilled in the art to which the present invention pertains will understand that the present invention can be implemented in other specific forms without changing the technical spirit or essential features thereof. In this regard, it should be understood that the examples described above are exemplary and not restrictive in all aspects. The scope of the present invention should be interpreted as including all changed or modified forms derived from the meaning and scope of the claims described below and equivalent concepts thereof rather than the detailed description above.

## Claims

1. A bifunctional glutamate N-acetyltransferase/amino acid acetyltransferase variant in which, from the N-terminus in the amino acid sequence of SEQ ID NO: 1, the amino acid corresponding to the 201st amino acid is substituted with phenylalanine, and the amino acid corresponding to the 355th amino acid is substituted with glutamic acid.

2. The bifunctional glutamate N-acetyltransferase/amino acid acetyltransferase variant according to claim 1, wherein the amino acid corresponding to the 201st amino acid is isoleucine, and the amino acid corresponding to the 355th amino acid is aspartic acid.

3. The bifunctional glutamate N-acetyltransferase/amino acid acetyltransferase variant according to claim 1, wherein the variant comprises an amino acid sequence having at least 90% sequence identity to the amino acid sequence of SEQ ID NO: 3.

4. A polynucleotide encoding the variant of any one claim of claim 1 to claim 3.

5. The polynucleotide according to claim 4, wherein the polynucleotide comprises a nucleic acid sequence having at least 90% sequence identity to the nucleic acid sequence of SEQ ID NO: 4.

6. A recombinant vector, comprising the polynucleotide of claim 4.

7. A microorganism of the genus *Corynebacterium,* comprising the variant of any one claim of claim 1 to claim 3, a polynucleotide encoding the variant, or a recombinant vector comprising the polynucleotide.

8. The microorganism of the genus *Corynebacterium* according to claim 7, wherein the microorganism has an increased production ability of glutamate family amino acids, as compared with a microorganism of the genus *Corynebacterium* comprising the polypeptide of SEQ ID NO: 1 or a polynucleotide encoding the same.

9. The microorganism of the genus *Corynebacterium* according to claim 8, wherein the glutamate family amino acids are any one or more selected from the group consisting of L-ornithine, L-citrulline, L-arginine and putrescine.

10. A method for producing glutamate family amino acids, comprising culturing a microorganism of the genus *Corynebacterium* comprising the variant of any one claim of claim 1 to claim 3, a polynucleotide encoding the variant, or a recombinant vector comprising the polynucleotide, in a medium, and
recovering glutamate family amino acids from the cultured microorganism, medium, or both of them.

11. The method for producing glutamate family amino acids according to claim 10, wherein the glutamate family amino acids are any one or more selected from the group consisting of L-ornithine, L-citrulline, L-arginine and putrescine.

12. A use of the microorganism of the genus *Corynebacterium* of claim 7, for producing glutamate family amino acids.

13. A composition, method, product, process, or use **characterized by** one or more elements disclosed in the present disclosure.

## INTERNATIONAL SEARCH REPORT

| | International application No. |
|---|---|
| | **PCT/KR2025/004328** |

**A. CLASSIFICATION OF SUBJECT MATTER**

**C12N 9/10**(2006.01)i; **C12N 15/77**(2006.01)i; **C12P 13/10**(2006.01)i

According to International Patent Classification (IPC) or to both national classification and IPC

**B. FIELDS SEARCHED**

Minimum documentation searched (classification system followed by classification symbols)

C12N 9/10(2006.01); C12N 1/20(2006.01); C12N 1/21(2006.01); C12N 15/77(2006.01); C12P 13/00(2006.01); C12P 13/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 이중 기능성 글루타메이트 N-아세틸트랜스퍼라제 /아미노산 아세틸트랜스퍼라제(bifunctional glutamate N-acetyltransferase/amino acid acetyltransferase), argJ, 치환(substitution), 변이체(variant), 아미노산(amino acid), 생산(production)

**C. DOCUMENTS CONSIDERED TO BE RELEVANT**

| Category* | Citation of document, with indication, where appropriate, of the relevant passages | Relevant to claim No. |
|---|---|---|
| A | KR 10-1813759 B1 (CJ CHEILJEDANG CORPORATION) 02 January 2018 (2018-01-02) See paragraphs [0052]-[0055] and [0060]; and claims 9-10, 16 and 22-23. | 1-12 |
| A | WO 2014-027702 A1 (AJINOMOTO CO., INC.) 20 February 2014 (2014-02-20) See entire document. | 1-12 |
| A | KR 10-1835935 B1 (CJ CHEILJEDANG CORPORATION) 12 March 2018 (2018-03-12) See entire document. | 1-12 |
| A | KR 10-1735935 B1 (CJ CHEILJEDANG CORPORATION) 16 May 2017 (2017-05-16) See entire document. | 1-12 |
| A | US 8741608 B2 (CLAES, Wilfried et al.) 03 June 2014 (2014-06-03) See entire document. | 1-12 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 July 2025** | **02 July 2025** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2025/004328** |

### C. DOCUMENTS CONSIDERED TO BE RELEVANT

| Category* | Citation of document, with indication, where appropriate, of the relevant passages | Relevant to claim No. |
| --- | --- | --- |
| A | US 6897048 B2 (SAKANYAN, Vehary et al.) 24 May 2005 (2005-05-24)<br>See entire document. | 1-12 |

Form PCT/ISA/210 (second sheet) (July 2022)

## INTERNATIONAL SEARCH REPORT

International application No.

**PCT/KR2025/004328**

| **Box No. I** | **Nucleotide and/or amino acid sequence(s) (Continuation of item 1.c of the first sheet)** |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/KR2025/004328**

| Box No. II | Observations where certain claims were found unsearchable (Continuation of item 2 of first sheet) |

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **13**
because they relate to parts of the international application that do not comply with the prescribed requirements to such an extent that no meaningful international search can be carried out, specifically:

The features (a composition, a method, a product, a process or use characterized by one or more elements disclosed in the present disclosure) of claim 13 make it difficult to clearly specify the scope of the invention, and thus a meaningful international search cannot be carried out.

3. ☐ Claims Nos.:
because they are dependent claims and are not drafted in accordance with the second and third sentences of Rule 6.4(a).

Form PCT/ISA/210 (continuation of first sheet) (July 2022)

# EP 4 756 018 A1

| INTERNATIONAL SEARCH REPORT | International application No. |
|---|---|
| Information on patent family members | PCT/KR2025/004328 |

| Patent document cited in search report | | | Publication date (day/month/year) | Patent family member(s) | | | Publication date (day/month/year) |
|---|---|---|---|---|---|---|---|
| KR | 10-1813759 | B1 | 02 January 2018 | CN | 107849576 | A | 27 March 2018 |
| | | | | EP | 3109318 | A1 | 28 December 2016 |
| | | | | EP | 3109318 | B1 | 05 June 2019 |
| | | | | JP | 2018-523989 | A | 30 August 2018 |
| | | | | JP | 6588575 | B2 | 09 October 2019 |
| | | | | KR | 10-2017-0000906 | A | 04 January 2017 |
| | | | | US | 11053525 | B2 | 06 July 2021 |
| | | | | US | 2018-0187222 | A1 | 05 July 2018 |
| | | | | WO | 2016-208854 | A1 | 29 December 2016 |
| WO | 2014-027702 | A1 | 20 February 2014 | None | | | |
| KR | 10-1835935 | B1 | 12 March 2018 | CN | 107002027 | A | 01 August 2017 |
| | | | | EP | 3153573 | A1 | 12 April 2017 |
| | | | | EP | 3153573 | B1 | 03 April 2019 |
| | | | | JP | 2017-518760 | A | 13 July 2017 |
| | | | | JP | 6476212 | B2 | 27 February 2019 |
| | | | | KR | 10-2016-0043890 | A | 22 April 2016 |
| | | | | US | 10626426 | B2 | 21 April 2020 |
| | | | | US | 2017-0226545 | A1 | 10 August 2017 |
| | | | | WO | 2016-060437 | A1 | 21 April 2016 |
| KR | 10-1735935 | B1 | 16 May 2017 | CN | 107922955 | A | 17 April 2018 |
| | | | | EP | 3141598 | A2 | 15 March 2017 |
| | | | | EP | 3141598 | B1 | 31 October 2018 |
| | | | | JP | 2018-520687 | A | 02 August 2018 |
| | | | | JP | 6543758 | B2 | 10 July 2019 |
| | | | | KR | 10-2017-0010960 | A | 02 February 2017 |
| | | | | US | 10640753 | B2 | 05 May 2020 |
| | | | | US | 10815464 | B2 | 27 October 2020 |
| | | | | US | 11268074 | B2 | 08 March 2022 |
| | | | | US | 11365400 | B2 | 21 June 2022 |
| | | | | US | 2018-0208909 | A1 | 26 July 2018 |
| | | | | US | 2020-0002686 | A1 | 02 January 2020 |
| | | | | US | 2020-0190486 | A1 | 18 June 2020 |
| | | | | US | 2021-0054347 | A1 | 25 February 2021 |
| | | | | WO | 2017-014532 | A1 | 26 January 2017 |
| US | 8741608 | B2 | 03 June 2014 | CN | 102947460 | A | 27 February 2013 |
| | | | | CN | 102947460 | B | 02 December 2015 |
| | | | | EP | 2553113 | A2 | 06 February 2013 |
| | | | | EP | 2553113 | B1 | 03 November 2021 |
| | | | | JP | 2013-524781 | A | 20 June 2013 |
| | | | | JP | 5855084 | B2 | 09 February 2016 |
| | | | | KR | 10-1782666 | B1 | 27 September 2017 |
| | | | | KR | 10-2013-0020774 | A | 28 February 2013 |
| | | | | US | 2011-0244529 | A1 | 06 October 2011 |
| | | | | US | 2014-0206068 | A1 | 24 July 2014 |
| | | | | US | 8951759 | B2 | 10 February 2015 |
| | | | | WO | 2011-124477 | A2 | 13 October 2011 |
| US | 6897048 | B2 | 24 May 2005 | CN | 1367244 | A | 04 September 2002 |
| | | | | CN | 1367244 | B | 26 May 2010 |
| | | | | EP | 1201758 | A1 | 02 May 2002 |

Form PCT/ISA/210 (patent family annex) (July 2022)

28

| Patent document cited in search report | Publication date (day/month/year) | Patent family member(s) | | Publication date (day/month/year) |
|---|---|---|---|---|
| | | EP | 1201758 B1 | 09 April 2008 |
| | | JP | 2002-315571 A | 29 October 2002 |
| | | JP | 4078515 B2 | 23 April 2008 |
| | | US | 2002-0098555 A1 | 25 July 2002 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020240055639
- US 9644009 B2
- KR 1020230048232
- US 7662943 B2
- US 10584338 B2
- US 10273491 B2
- KR 101632642

### Non-patent literature cited in the description

- **PEARSON et al.** *Proc. Natl. Acad. Sci. USA*, 1988, vol. 85, 2444
- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **DEVEREUX, J. et al.** *Nucleic Acids Research*, 1984, vol. 12, 387
- **ATSCHUL, [S.] [F.** *J MOLEC BIOL*, 1990, vol. 215, 403
- Guide to Huge Computers. Academic Press, 1994
- **CARILLO**. *SIAM J Applied Math*, 1988, vol. 48, 1073
- **NEEDLEMAN et al.** *J Mol Biol.*, 1970, vol. 48, 443
- **SMITH** ; **WATERMAN**. *Adv. Appl. Math*, 1981, vol. 2, 482
- **GRIBSKOV et al.** *Nucl. Acids Res.*, 1986, vol. 14, 6745
- Atlas Of Protein Sequence And Structure. National Biomedical Research Foundation, 1979, 353-358
- **J. SAMBROOK et al.** Molecular Cloning, A Laboratory Manual. Cold Spring Harbor Laboratory press, 1989
- **F.M. AUSUBEL et al.** Current Protocols in Molecular Biology. John Wiley & Sons, Inc
- **NAKASHIMA N et al.** Bacterial cellular engineering by genome editing and gene silencing.. *Int J Mol Sci*, 2014, vol. 15 (2), 2773-2793
- **SAMBROOK et al.** *Molecular Cloning*, 2012
- **WEINTRAUB, H. et al.** Antisense-RNA as a molecular tool for genetic analysis. *Reviews - Trends in Genetics*, 1986, vol. 1 (1)
- **SITNICKA et al.** *Functional Analysis of Genes. Advances in Cell Biology*, 2010, vol. 2, 1-16
- Manual of Methods for General Bacteriology. American Society for Bacteriology, 1981
- *Appl. Microbiol. Biotechnol*, 1999, vol. 52, 541-545